# EUROPÄISCHE PATENTSCHRIFT

(11) **EP 4 523 630 B1**
(45) Veröffentlichungstag und Bekanntmachung des Hinweises auf die Patenterteilung: **18.02.2026**
(21) Anmeldenummer: 23197481.7
(22) Anmeldetag: 14.09.2023
(51) Int. Cl.: A61B 6/03

(54) **VORHERSAGE DER DIAGNOSESICHERHEIT**
PREDICTING DIAGNOSTIC SAFETY
PRÉDICTION DE SÉCURITÉ DE DIAGNOSTIC

(43) Veröffentlichungstag der Anmeldung: 19.03.2025
(73) Patentinhaber: Siemens Healthineers AG, 91301 Forchheim (DE)
(72) Erfinder: Roser, Philipp, 91052 Erlangen (DE)
(74) Vertreter: Siemens Healthineers Patent Attorneys

(56) Entgegenhaltungen:
- US-A1- 2013 108 130
- PATRIK NOWIK ET AL: "Quality control of CT systems by automated monitoring of key performance indicators: a two-year study", JOURNAL OF APPLIED CLINICAL MEDICAL PHYSICS, vol. 16, no. 4, 1 July 2015 (2015-07-01), US, pages 254 - 265, XP055459487, ISSN: 1526-9914, DOI: 10.1120/jacmp.v16i4.5469
- VERDUN F R ET AL: "Image quality in CT: From physical measurements to model observers", PHYSICA MEDICA, ACTA MEDICA EDIZIONI E CONGRESSI, ROME, IT, vol. 31, no. 8, 12 October 2015 (2015-10-12), pages 823 - 843, XP029350407, ISSN: 1120-1797, DOI: 10.1016/J.EJMP.2015.08.007

## Beschreibung

Die vorliegende Erfindung betrifft ein Verfahren zum Betreiben einer Röntgenbildgebungsvorrichtung durch Eingeben eines Objektwerts betreffend ein zu durchleuchtendes Objekt in die Röntgenbildgebungsvorrichtung und Einstellen eines Röntgenparameters der Röntgenbildgebungsvorrichtung. Darüber hinaus betrifft die vorliegende Erfindung eine Röntgenbildgebungsvorrichtung.

Kegelstrahl-Computertomographen (CBCT) und Flachdetektor-Angiographieanlagen werden häufig für interventionelle Zwecke eingesetzt, z.B. für die intraoperative Verfolgung von Geräten oder die Führung solcher Geräte. Die Qualität der Aufnahmen ist in der Regel gegenüber z.B. Spiral-Computertomographen deutlich reduziert. Die Verwendung der Kegelstrahl-Computertomographen für voxelbasierte quantitative oder zumindest qualitative diagnostische Zwecke wird jedoch zunehmend wünschenswert. Gegebenenfalls soll mit solchen Anlagen eine verbesserte Gewebe- beziehungsweise Materialunterscheidung ermöglicht werden (z.B. Dual energy material decomposition).

Diagnostische Anwendungen, wie z.B. die Dual-Energy-Diagnostic, hängen oft stark von der Genauigkeit der rekonstruierten Hounsfield-Einheiten (HUs) ab. Die Hounsfield-Einheiten ergeben sich aus der Hounsfield-Skala, mit der in der Computertomographie eine Abschwächung der Röntgenstrahlung in Objekten beschrieben wird. Mit den Hounsfield-Einheiten können Graustufenbilder dargestellt werden. Sie eignen sich zum Unterscheiden von Gewebearten. Dazu ist jedoch eine gewisse HU-Treue erforderlich. Bei CBCT ist diese HU-Treue aufgrund verschiedener Effekte, z.B. Strahlenhärtung, Streuung, unvollständige Daten und so weiter, im Allgemeinen nicht gegeben.

Dennoch reicht in vielen Fällen die HU-Treue aus, um diagnostische oder quantitative Algorithmen sicher anwenden zu können. Vor dem eigentlichen CBCT-Scan ist jedoch nicht bekannt, ob die erforderliche Mindest-HU-Treue erreicht werden kann. Auch nach der Aufnahme beruht die Einschätzung der diagnostischen Sicherheit in der Regel auf der Erfahrung und Intention des Radiologen.

Die US 2013/108130 A1 beschreibt ein Röntgen-CT-Gerät mit einer Schätzvorrichtung zur Bestimmung einer Merkmalsgröße, die mit der Bildqualität oder dem Pixelwert eines durch CT-Scan erzeugten Bildes korreliert.

Die Aufgabe der vorliegenden Erfindung besteht darin, den Bediener einer Röntgenbildgebungsvorrichtung im Hinblick auf eine Diagnose besser zu unterstützen.

Erfindungsgemäß wird diese Aufgabe durch ein Verfahren und eine Röntgenbildgebungsvorrichtung gemäß den unabhängigen Ansprüchen gelöst. Vorteilhafte Weiterbildungen der Erfindung ergeben sich aus den Unteransprüchen.

Entsprechend der vorliegenden Erfindung wird demnach ein Verfahren zum Betreiben einer Röntgenbildgebungsvorrichtung bereitgestellt. Bei der Röntgenbildgebungsvorrichtung kann es sich beispielsweise um den eingangs erwähnten Kegelstrahl-Computertomographen (CBCT) beziehungsweise eine Flachdetektor-Angiographieanlage handeln. Das Verfahren beschreibt Teilschritte für den Betrieb der Röntgenbildgebungsvorrichtung.

Es erfolgt ein Eingeben eines Objektwerts betreffend ein zu durchleuchtendes Objekt in die Röntgenbildgebungsvorrichtung. Das zu durchleuchtende Objekt kann ein Patient oder ein technischer Gegenstand sein, von dem ein Röntgenbild gewonnen werden muss. Der Objektwert betrifft das zu durchleuchtende Objekt und beschreibt dieses. Beispielsweise handelt es sich bei dem Objektwert um die Patientengröße, das Patientengewicht, die Materialstärke eines technischen Gegenstands, die Materialart eines technischen Gegenstands und dergleichen. Dieser Objektwert muss in die Röntgenbildgebungsvorrichtung eingegeben werden. Dazu ist gegebenenfalls eine entsprechende Schnittstelle vorzusehen.

In einem weiteren Schritt erfolgt ein Einstellen eines Röntgenparameters der Röntgenbildgebungsvorrichtung. Auch dieses Einstellen kann an einer entsprechenden Schnittstelle **z.B.** einer Steuerungseinrichtung der Röntgenbildgebungsvorrichtung erfolgen. Röntgenparameter kann beispielsweise die Röntgenspannung, die Aufnahmezeit und so weiter sein.

In einem weiteren Schritt erfolgt ein Ermitteln eines Konfidenzwerts bezüglich einer Qualität eines aufzunehmenden oder zu rekonstruierenden Röntgenbilds mit Hilfe eines Modells auf der Basis des Objektwerts und des Röntgenparameters. Der Konfidenzwert kann auch als diagnostischer Vertrauenswert bezeichnet werden, da er angibt, mit welcher Sicherheit eine bestimmte Qualität eines Röntgenbilds erreicht werden kann. Die Qualität kann beispielsweise dadurch beurteilt werden, inwieweit sich die Quantitätswerte des Röntgenbilds in einem gewissen Toleranzbereich befinden. Der Konfidenzwert dient also zur Vorhersage der Qualität des aufzunehmenden oder zu rekonstruierenden Röntgenbilds. Bei dem Röntgenbild kann es sich um eine 2D-Aufnahme oder eine 3D-Rekonstruktion mehrerer 2D-Aufnahmen handeln.

Die Ermittlung des Konfidenzwerts erfolgt mit Hilfe eines Modells, das mit dem (mindestens einen) Objektwert und dem (mindestens einen) Röntgenparameter variierbar ist. Betrifft der Objektwert beispielsweise die anatomische Region und gibt an, dass es sich bei dem zu durchleuchtenden Bereich um einen Abdomenabschnitt handelt, so wird das Modell den Konfidenzwert beispielsweise herabsetzen, da die Röntgenstreuung im Abdomen verhältnismäßig groß ist und damit die diagnostische Sicherheit abnimmt. Umgekehrt wird das Modell den Konfidenzwert gegebenenfalls erhöhen, wenn die Röntgenspannung erhöht wird und damit unter Umständen der Kontrast erhöht wird.

Schließlich erfolgt in einem weiteren Schritt ein Bereitstellen des Konfidenzwerts für einen Bediener oder zum Steuern der Röntgenbildgebungsvorrichtung. Beispielsweise kann ein Gesamtkonfidenzwert für ein gesamtes aufzunehmendes oder zu rekonstruierendes Röntgenbild als Zahl oder Farbe auf einem Monitor bereitgestellt werden. Alternativ kann auch eine Konfidenzwertverteilung (als "Konfidenzwert") beispielsweise als 2D- oder 3D-Graphik dargestellt werden. Dabei kann für jedes Pixel oder Voxel ein jeweiliger individueller Konfidenzwert berechnet werden. Die Darstellung des Konfidenzwerts beziehungsweise der Konfidenzwerte auf einem Monitor kann dem Bediener eine Diagnose erleichtern, da er beispielsweise erfährt, dass es sich mit 90%iger Wahrscheinlichkeit um Lebergewebe handelt. Alternativ oder zusätzlich kann der Konfidenzwert beziehungsweise können die Konfidenzwerte auch zum Steuern der Röntgenbildgebungsvorrichtung bereitgestellt werden. Dazu ist gegebenenfalls eine Steuerungseinrichtung notwendig, um die Konfidenzwerte zu speichern und für das Steuern der Röntgenbildgebungsvorrichtung auszulesen. Gegebenenfalls werden die Konfidenzwerte an einem entsprechenden Steuereingang der Röntgenbildgebungsvorrichtung bereitgestellt.

In vorteilhafter Weise kann somit ein Empfehlungssystem beziehungsweise -verfahren bereitgestellt werden, das die mögliche diagnostische Sicherheit bei einem bestimmten Bildgebungsprotokoll und/oder einer Anwendung vorhersagt. Weiter vorteilhaft ist, dass die Erfindung für Dual-Energy-Anwendungen eingesetzt werden kann, sie ist aber nicht darauf beschränkt.

Erfindungsgemäß ist vorgesehen, dass sich der Konfidenzwert auf einen rekonstruierten Hounsfield-Wert bezieht. Ein 3D-Röntgenbild lässt sich beispielsweise aus mehreren Röntgenaufnahmen rekonstruieren. Ebenso kann ein virtuelles 3D-Bild aus zu erwartenden Röntgenbildern rekonstruiert werden, die sich mit Hilfe des Modells aus dem mindestens einen Objekt und dem mindestens einen Röntgenparameter ergeben würden. Das rekonstruierte 3D-Bild wäre ein 3D-Datensatz aus Hounsfield-Werten. Der Konfidenzwert kann sich nun auf die Gesamtheit der Hounsfield-Werte, nur einen Teilbereich der Hounsfield-Werte oder nur auf einen einzelnen Hounsfield-Wert beziehen.

Bei einem weiteren Ausführungsbeispiel ist der Konfidenzwert einer von vielen Konfidenzwerten, von denen jeder für ein jeweiliges Pixel oder Voxel des aufzunehmenden oder zu rekonstruierenden Röntgenbilds ermittelt wird. Es wird also für jedes Pixel oder Voxel ein Konfidenzwert vorhergesagt. Daraus lässt sich eine sogenannte "Heat map" erstellen, aus der beispielsweise durch Farbwerte ersichtlich ist, in welchen Bildbereichen niedrige Konfidenzwerte vorliegen, was auf schlechte zu erwartende Bildqualität hindeutet. Ein Bediener kann dann, beispielsweise mit der Einstellung der Röntgenbildgebungsvorrichtung, entsprechend entgegenwirken, sofern er an einem bestimmten Bildbereich besonders interessiert ist.

Gemäß einem weiteren Ausführungsbeispiel ist vorgesehen, dass ein Wertebereich von Hounsfield-Werten einem Gewebetyp oder Objektbereich zugeordnet wird, für den Wertebereich der Konfidenzwert ermittelt wird und somit mittelbar über den Wertebereich dem Gewebetyp oder Objektbereich der Konfidenzwert zugeordnet wird. Soll beispielsweise Lebergewebe nachgewiesen werden, so liegen dessen Hounsfield-Werte beispielsweise bei einem Patienten bestimmter Größe und bestimmten Gewichts in einem bekannten Wertebereich. Für diesen Wertebereich kann mittels des Modells der Konfidenzwert ermittelt werden. Indirekt wird also dem Gewebetyp beziehungsweise Objektbereich ein Konfidenzwert zugeordnet. Mit dem Konfidenzwert erhält der Bediener einen Anhaltspunkt darüber, wie sicher er bei dem Patienten (beschrieben durch entsprechende Objektwerte) und bei der gewählten Einstellung der Röntgenparameter der Röntgenbildgebungsvorrichtung das Lebergewebe identifizieren kann. In ähnlicher Weise kann beispielsweise für Blut ein Konfidenzwert ermittelt werden und der Bediener kann die Röntgenparameter der Röntgenbildgebungsvorrichtung ändern, wenn ihm der zugehörige Konfidenzwert zu hoch oder zu niedrig ist.

Analog kann der Konfidenzwert auch für einen Bereich des Objekts oder einen Bereich des Bilds (Objektbereich) ermittelt werden. Beispielsweise ist es wichtig, in einem Bereich um die Wirbelsäule eine hohe Qualität zu erzielen. In diesem Fall sollte für den gewählten Bereich ein hoher Konfidenzwert vorliegen. Dies kann der Bediener beispielsweise mit der Wahl der Röntgenparameter beeinflussen. Dabei kann in dem gewählten Bereich beispielsweise der Konfidenzwert durch eine Mittelwertbildung oder ein Medianwertbildung aus mehreren Konfidenzwerten ermittelt werden.

Gemäß einem weiteren Ausführungsbeispiel ist der Konfidenzwert ein für eine jeweilige Schicht des zu rekonstruierenden Röntgenbilds standardisierter Wert. Beispielsweise können für unterschiedliche Schichten des Objekts nur unterschiedliche Konfidenzwerte erreicht werden. Damit läge eine Inhomogenität in Bezug auf die Schichten vor. Diese Inhomogenität kann durch einen Homogenitätsindex ausgedrückt werden. Die Homogenität lässt sich beispielsweise dadurch erhöhen, dass für die einzelnen Schichten standardisierte beziehungsweise normierte Konfidenzwerte verwendet werden. Hinsichtlich des Homogenitätsindexes kann verwiesen werden auf: Nowik P, Bujila R, Poludniowski G, Fransson A. Quality control of CT systems by automated monitoring of key performance indicators: a twoyear study. J Appl Clin Med Phys. 2015 Jul 8;16(4):254-265. doi: 10.1120/jacmp.v16i4.5469. PMID: 26219012; PMCID: PMC5690007. Demnach ist die KPI-Homogenität (key performance indicators) definiert als die maximale Differenz der mittleren CT-Zahl (bzw. Hounsfield Unit) zwischen den einzelnen ROIs (region of interest). Der Artikel beschreibt auch die Uniformität als weiteren Schlüsselindex. Der Einheitlichkeits- bzw. Uniformitäts-KPI ist ein Test, der die Leistung des CT-Scanners bei der Rekonstruktion eines einheitlichen Bildes bewertet.

Entsprechend einem weiteren Ausführungsbeispiel ist vorgesehen, dass das Bereitstellen des Konfidenzwerts in einer Karte erfolgt, in der an jedem Pixelort oder Voxelort ein entsprechender Konfidenzwert dargestellt wird. Es wird also die oben angesprochene Heat map entwickelt, die ortsgenau jeweilige Konfidenzwerte widergibt. Die einzelnen Konfidenzwerte der Pixel oder Voxel werden also nicht nur dazu verwendet, gegebenenfalls einen Mittelwert zu berechnen, sondern die einzelnen Konfidenzwerte werden auch einzeln in der jeweiligen Darstellung zweidimensional oder dreidimensional widergegeben. Damit kann sich der Bediener für die Einstellung der Röntgenbildgebungsvorrichtung genauer orientieren.

In einem weiteren Ausführungsbeispiel wird bei dem Ermitteln des Konfidenzwerts ein Qualitätsmaß bezüglich einer Rekonstruierbarkeit eines Voxel in Abhängigkeit von einer Detektortrajektorie der Röntgenbildgebungsvorrichtung berücksichtigt. Ist beispielsweise ein Voxel bei einer bestimmten CBCTbeziehungsweise Detektortrajektorie kaum rekonstruierbar, so kann der Konfidenzwert entsprechend herabgesetzt werden. Umgekehrt kann bei guter Rekonstruierbarkeit der Konfidenzwert für ein Voxel entsprechend heraufgesetzt werden. Die Rekonstruierbarkeit kann voxelindividuell berücksichtigt werden.

Bei einem weiteren Ausführungsbeispiel ist vorgesehen, dass das zu durchleuchtende Objekt ein Patient ist und der Objektwert mindestens einen der folgenden Werte betrifft: Größe, Gewicht, Geschlecht, Wasserwert oder anatomische Region des Patienten. Der Objektwert beschreibt hier also den Patienten. Diese Patientenwerte werden zur Ermittlung der Konfidenzwerte genutzt. Es können einer oder mehrere dieser Werte verwendet werden. Beispielsweise beeinflussen Größe und Gewicht direkt das Röntgenergebnis, da die Qualität der Röntgenbilder von der jeweiligen Durchleuchtungsstrecke abhängen. Ähnliches gilt beispielsweise für die anatomische Region des Patienten, denn im Abdomen sind beispielsweise mehr Streuungen zu erwarten als im Kopfbereich.

In einem anderen Ausführungsbeispiel umfasst der Objektwert einen 3D-Datensatz, der von dem Objekt gewonnen wird, und ein jeweiliger Konfidenzwert für jedes Voxel des 3D-Datensatzes wird ermittelt. Beispielsweise wird präoperativ oder auch intraoperativ ein 3D-Datensatz von einem Patienten gewonnen, welcher für die Vorhersage weiterer aufzunehmender Röntgenbilder genutzt wird. So kann der 3D-Datensatz dazu genutzt werden, für jedes seiner Voxel mit Hilfe des Modells einen Konfidenzwert zu bestimmen.

Bei einer weiteren Ausführungsform kann vorgesehen sein, dass bei dem Ermitteln des Konfidenzwerts ein statistisches Objektmodell zu Grunde gelegt wird, das mit dem (mindestens einen) Objektwert parametrisiert wird. Beispielsweise kann es sich bei dem statistischen Objektmodell um ein statistisches Patientenformmodell oder ein statistisches Patientenorganmodell oder dergleichen handeln. Mit diesen Modellen und beispielsweise Größen- und Gewichtsangaben, mit denen die Modelle parametrisiert werden, können die Röntgenergebnisse noch präziser vorhergesagt werden. Beispielsweise kann die Patientenform mit einem Patientenformmodell sehr genau modelliert werden, wenn dieses entsprechend mit Größe und/oder Gewicht des Patienten parametrisiert ist. Ähnliches gilt für Organe des Patienten, deren Gestalt sehr genau mit einem statistischen Patientenorganmodell beschrieben werden kann, das mit Größe, Gewicht und/oder Geschlecht des Patienten parametrisiert ist.

Bei einem weiteren Ausführungsbeispiel ist vorgesehen, dass der Röntgenparameter der Röntgenbildgebungsvorrichtung mindestens eine der folgenden Werte betrifft: Röhrenspannung, Röhrenstrom, Detektorwinkel oder Detektorposition. Die Röntgenbildgebungsvorrichtung wird mit diesen Röntgenparametern eingestellt, welche das Durchleuchtungsergebnis unmittelbar beeinflussen. So beeinflusst beispielsweise die Röhrenspannung die Energieverteilung der Röntgenstrahlung. Je höher die Röhrenspannung ist, desto härter wird die Strahlung, da der Anteil an hochenergetischen Photonen zunimmt. Je härter die Röntgenstrahlung ist, desto eher durchdringt sie Materie. Daher kann der materialabhängige Kontrast über die Röhrenspannung eingestellt werden. Über den Röhren- beziehungsweise Heizstrom kann die Intensität der Röntgenstrahlung beeinflusst werden. Mit diesen Größen kann somit die Helligkeit und der Kontrast des Röntgenbilds und damit auch der Konfidenzwert beeinflusst werden. In ähnlicher Weise haben natürlich auch Detektorwinkel und Detektorposition sowie Winkel und Position der Strahlungsquelle beziehungsweise deren Trajektorien Einfluss auf die Röntgenbilder. Dementsprechend können auch Rekonstruktionen und deren Konfidenzwerte davon betroffen sein.

In einem weiteren Ausführungsbeispiel ist vorgesehen, dass das Ermitteln des Konfidenzwerts mit Hilfe einer Look-Up-Tabelle, einer parametrierbaren Funktion oder eines Algorithmus zum maschinellen Lernen erfolgt. Über die Look-Up-Tabelle ist eine einfache Wertezuordnung möglich. Mit einer parametrierbaren Funktion kann die Zuordnung mit Hilfe der Parameter variiert werden. Größte Flexibilität erhält man mit einem Algorithmus zum maschinellen Lernen, der für die Zuordnung des Konfidenzwerts verantwortlich ist. Beispielsweise kann das maschinelle Lernen mit Hilfe eines neuronalen Netzes erfolgen. Eingangsgrößen können dort die oben genannten Objektwerte und gegebenenfalls auch ein 3D-Datensatz des Objekts sein. Darüber hinaus können auch die Röntgenparameter Eingangsgrößen für das neuronale Netz sein. Ausgangsgrößen des neuronalen Netzes beziehungsweise des Algorithmus zum maschinellen Lernen könnten entsprechend die Konfidenzwerte sein.

Gemäß einem weiteren Ausführungsbeispiel wird in Abhängigkeit von dem bereitgestellten Konfidenzwert ein Parameter der Röntgenbildgebungsvorrichtung automatisch eingestellt. Der bereitgestellte Konfidenzwert wird somit zur Steuerung der Röntgenbildgebungsvorrichtung verwendet. Dabei wird der Konfidenzwert beziehungsweise die Vielzahl der Konfidenzwerte dazu benutzt, einen Röntgenparameter automatisch zu verändern beziehungsweise einzustellen. Beispielsweise kann die Röhrenspannung oder ein Verhältnis von Röhrenspannung und Röhrenstrom automatisch verändert werden, wenn der ermittelte Konfidenzwert eine vorbestimmte Größe nicht erreicht. Die Röntgenbildgebungsvorrichtung schlägt damit einen neuen Parameterwert vor, der für eine vorzunehmende Aufnahme eingestellt wird. Falls dann beispielsweise der Bediener mit dem Vorschlag einverstanden ist, kann die Röntgenaufnahme mit dem vorgeschlagenen Parameterwert durchgeführt werden.

Die oben genannte Aufgabe wird erfindungsgemäß auch gelöst durch eine Röntgenbildgebungsvorrichtung mit
- einer ersten Schnittstelleneinrichtung zum Eingeben eines Objektwerts betreffend ein zu durchleuchtendes Objekt in die Steuerungseinrichtung und zum Einstellen eines Röntgenparameters der Röntgenbildgebungsvorrichtung,
- einer Recheneinrichtung ausgebildet zum Ermitteln eines Konfidenzwerts bezüglich einer Qualität eines aufzunehmenden Röntgenbilds mit Hilfe eines Modells auf der Basis des Objektwerts und des Röntgenparameters und
- einer zweiten Schnittstelleneinrichtung zum Bereitstellen des Konfidenzwerts für einen Bediener oder (für eine Steuereinrichtung) zum Steuern der Röntgenbildgebungsvorrichtung.

Die erste Schnittstelleneinrichtung zum Eingeben des Objektbeziehungsweise Patientenwerts und zum Einstellen des Röntgenparameters kann eine Datenschnittstelle umfassen, in die entsprechende Daten elektronisch eingegeben werden können. Darüber hinaus kann diese erste Schnittstelleneinrichtung auch Einstellelemente wie etwa ein Stellrad, einen Joystick oder einen Touchscreen aufweisen. Die Recheneinrichtung zum Ermitteln des Konfidenzwerts kann mindestens einen Prozessor und mindestens ein Speicherelement aufweisen. Die zweite Schnittstelleneinrichtung zum Bereitstellen des Konfidenzwerts kann einen Monitor aufweisen, mit dem der Konfidenzwert oder eine Vielzahl von Konfidenzwerten optisch dargestellt werden. Falls der beziehungsweise die Konfidenzwerte zum Steuern der Röntgenbildgebungsvorrichtung direkt verwendet werden, können die Konfidenzwerte auch von der zweiten Schnittstelleneinrichtung elektronisch für die Röntgenbildgebungsvorrichtung bereitgestellt werden. Dazu kann die zweite Schnittstelleneinrichtung beispielsweise einen Dateneingang und gegebenenfalls eine Speichereinrichtung aufweisen. Die erste und zweite Schnittstelleneinrichtung können auch ineinander integriert sein.

In einem Ausführungsbeispiel ist die Röntgenbildgebungsvorrichtung als Kegelstrahl-Computertomographie-Gerät oder als (Flachdetektor-) Angiographiegerät ausgebildet. Alternativ kann die Röntgenbildgebungsvorrichtung aber auch als Spiral-CT oder anderes Röntgengerät realisiert sein.

Die oben in Zusammenhang mit dem erfindungsgemäßen Verfahren genannten Vorteile und Variationsmöglichkeiten gelten sinngemäß auch für die erfindungsgemäße Röntgenbildgebungsvorrichtung. Dementsprechend können die in Zusammenhang mit dem Verfahren geschilderten Merkmale als funktionelle Merkmale der Röntgenbildgebungsvorrichtung angesehen werden.

Die obige Aufgabe wird erfindungsgemäß auch gelöst durch ein Computerprogramm, das Befehle aufweist, welche, wenn sie in einem Prozessor der Röntgenbildgebungsvorrichtung obiger Art ausgeführt werden, diese veranlassen, das oben beschriebene Verfahren auszuführen.

Für Anwendungsfälle oder Anwendungssituationen, die sich bei dem Verfahren ergeben können und die hier nicht explizit beschrieben sind, kann vorgesehen sein, dass gemäß dem Verfahren eine Fehlermeldung und/oder eine Aufforderung zur Eingabe einer Nutzerrückmeldung ausgegeben und/oder eine Standardeinstellung und/oder ein vorbestimmter Initialzustand eingestellt wird.

Unabhängig vom grammatikalischen Geschlecht eines bestimmten Begriffes sind Personen mit männlicher, weiblicher oder anderer Geschlechteridentität mit umfasst.

Die vorliegende Erfindung wird nun anhand der beigefügten Zeichnungen näher erläutert, in denen zeigen:
- FIG 1: ein bekanntes C-Bogen-Angiographiesystem mit einem Industrieroboter als Tragvorrichtung und
- FIG 2: einen schematischen Verfahrensablauf eines Ausführungsbeispiels der vorliegenden Erfindung.

Die nachfolgend näher geschilderten Ausführungsbeispiele stellen bevorzugte Ausführungsformen der vorliegenden Erfindung dar.

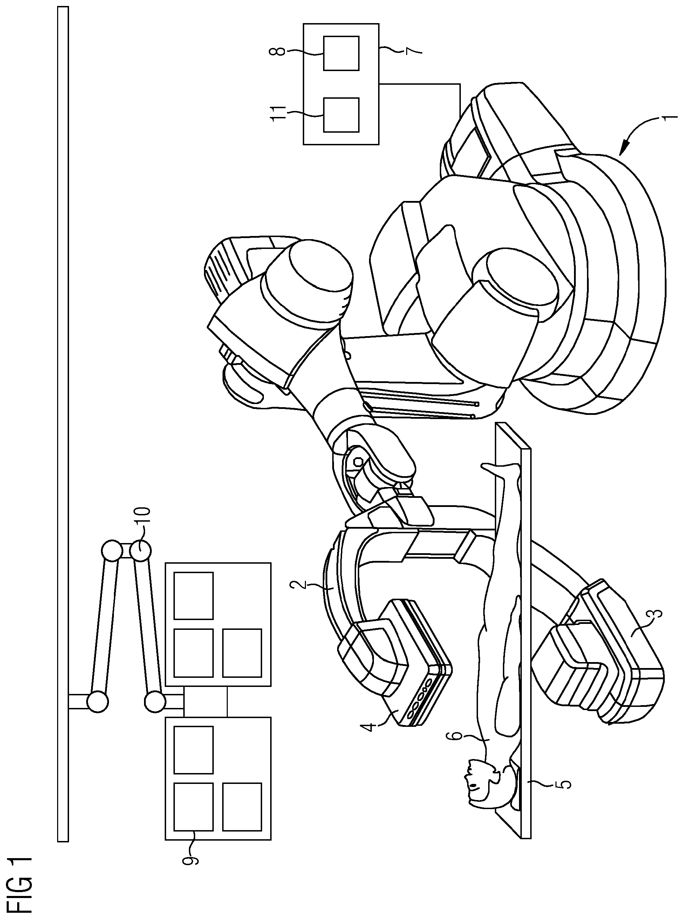

Die FIG 1 zeigt ein als Beispiel dargestelltes monoplanes Röntgensystem mit einem von einem Ständer 1 in Form eines sechsachsigen Industrie- oder Knickarmroboters gehaltenen C-Bogen 2, an dessen Enden eine Röntgenstrahlungsquelle, beispielsweise ein Röntgenstrahler 3 mit Röntgenröhre und Kollimator, und ein Röntgenbilddetektor 4 als Bildaufnahmeeinheit angebracht sind. Die Realisierung der Röntgendiagnostikeinrichtung ist nicht auf den Industrieroboter angewiesen. Es können auch übliche C-Bogen-Geräte Verwendung finden.

Im Strahlengang des Röntgenstrahlers 3 befindet sich auf einer Tischplatte 5 eines Patientenlagerungstisches ein zu untersuchender Patient 6 oder ein technischer Gegenstand als Untersuchungsobjekt. An der Röntgendiagnostikeinrichtung ist eine Systemsteuerungseinheit 7 mit einer Recheneinrichtung 8 zur Bildverarbeitung angeschlossen, die die Bildsignale des Röntgenbilddetektors 4 empfängt und verarbeitet (Bedienelemente sind beispielsweise nicht dargestellt). Die Röntgenbilder können dann auf Displays einer Monitorampel 9 betrachtet werden. Die Monitorampel 9 kann mittels eines deckenmontierten, längs verfahrbaren, schwenk-, dreh- und höhenverstellbaren Trägersystems 10 mit Ausleger und absenkbarem Tragarm gehalten werden. In der Systemsteuerungseinheit 7 ist weiterhin ein Empfehlungssystem 11 zum Ermitteln eines Konfidenzwerts vorgesehen.

Bei dem nachfolgenden Ausführungsbeispiel wird ein Empfehlungssystem bereitgestellt, das die diagnostische Sicherheit eines bestimmten Bildgebungsprotokolls und/oder einer Anwendung vorhersagt. Obwohl die Erfindung am Beispiel von Dual-Energy-Anwendungen erläutert wird, ist sie nicht auf die Verwendung von Dual-Energy beschränkt.

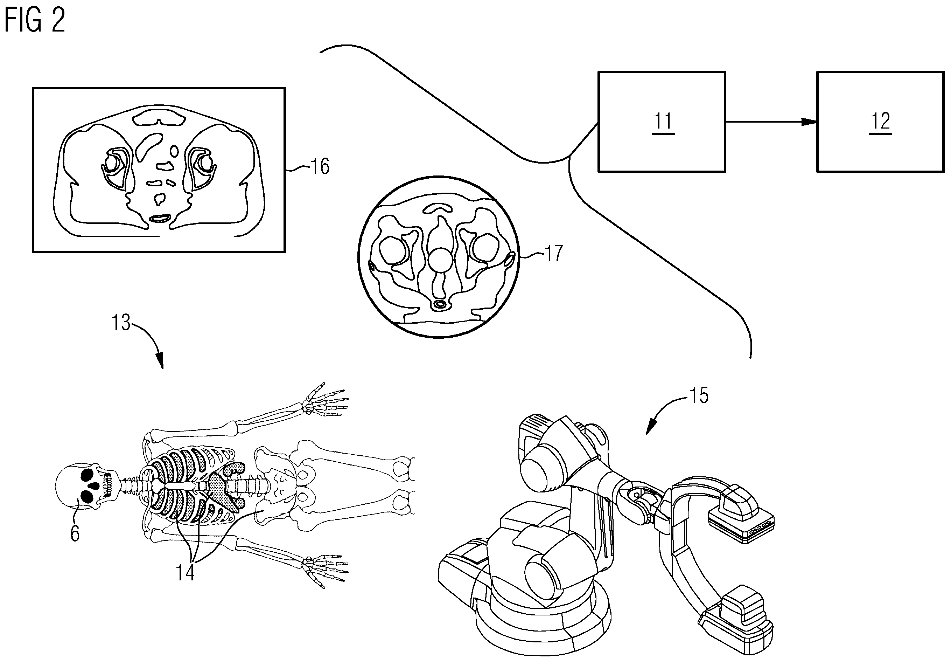

Die Erfindung basiert auf der Idee, einen diagnostischen Konfidenzwert aus mindestens einem Objektwert, der ein zu durchleuchtendes Objekt beschreibt, und mindestens einem Röntgenparameter, mit dem eine Röntgenbildgebungsvorrichtung eingestellt wird, mit Hilfe eines Empfehlungssystems 11 vorherzusagen beziehungsweise auszugeben. Im Folgenden werden verschiedene Implementationen des diagnostischen Konfidenzwerts 12, der Eingangsparameter (Objektwert 13, Röntgenparameter 15) und des Empfehlungssystems 11 dargestellt (vgl. FIG 2).

Der diagnostische Konfidenzwert 12 stellt einen Vertrauenswert dar, der die Qualität eines einzelnen Pixels, eines einzelnen Voxel oder einer Pixelgruppe beziehungsweise Voxelgruppe widergeben soll. Der Konfidenzwert wird mit Hilfe eines Modells berechnet und kann beispielsweise Werte im Bereich von 0-1 oder 0% bis 100% besitzen. Dabei steht 1 für die höchste Konfidenz, wenn z.B. ein nahezu artefaktfreier CT-Scan erwartet wird.

Der Konfidenzwert 12 kann aber auch einen sogenannten HU-Treue-Wert (Hounsfield fidelity value) widerspiegeln. Der HU-Treue-Wert kann beispielsweise die erwartete Sicherheit beziehungsweise Unsicherheit der rekonstruierten HU-Werte angeben. Optional kann der HU-Treue-Wert in Abhängigkeit von einem Gewebetyp oder für bestimmte anatomische Bereiche angegeben werden.

Der Konfidenzwert 12 kann aber auch ein Homogenitätsindexwert sein, welcher einen standardisierten Wert der HU-Treue darstellt (siehe oben).

Darüber hinaus kann der Konfidenzwert als sogenannte "Heat map" realisiert sein. Dies bedeutet, dass der Konfidenzwert als zweidimensionale oder dreidimensionale Werteverteilung realisiert ist. Es erfolgt hierbei eine Schätzung der oben genannten Werte pro Pixel oder Voxel für das rekonstruierbare Volumen.

In den Konfidenzwert 12 können aber auch eine oder mehrere Bedingungen beispielsweise zusätzlich zu dem HU-Treue-Wert einfließen. Beispielsweise kann das Empfehlungssystem 11 (vergleiche FIG 2) zum Schätzen des Konfidenzwerts 12 eine Vollständigkeitsbedingung heranziehen. Demnach kann für jedes Pixel oder Voxel bestimmt werden, wie gut beziehungsweise vollständig es bei einer bestimmten CBCT-Trajektorie rekonstruiert werden kann. Diese Rekonstruierbarkeit kann für die Schätzung des Konfidenzwerts ein Zusatzmerkmal sein, aber ebenso auch alleiniges Merkmal.

Das Empfehlungssystem 11 benötigt zur Schätzung eines oder mehrerer Konfidenzwerte beziehungsweise einer Konfidenzwertverteilung 12 mehrere Eingabeparameter, wie FIG 2 verdeutlicht. Zum einen benötigt das Empfehlungssystem mindestens einen Objektwert 13, der das Objekt, z.B. den Patienten 6, beschreibt. Bei dem Objektwert kann es sich beispielsweise um die Größe, das Gewicht, das Geschlecht, einen Wasserwert, eine anatomische Region 14 des Patienten 6 handeln. Prinzipiell kann der Objektwert auch ein Objektvektor aus mehreren der genannten Größen sein. Als anatomische Regionen kämen beispielsweise der Schädel, die Lunge, die Leber, die Nieren, das Becken et cetera infrage. All diese Regionen haben ihre Eigenheiten bei der Rekonstruktion, die sich auf den Konfidenzwert auswirken können.

Darüber hinaus benötigt das Empfehlungssystem 11 für das Ermitteln des Konfidenzwerts 12 mindestens einen Röntgenparameter 15, mit dem die Röntgenbildgebungsvorrichtung eingestellt wird. Auch bei dem Röntgenparameter kann es sich um einen Röntgenvektor handeln, der aus mehreren Parametern zusammengesetzt ist. Ein Aufnahmeprotokoll der Röntgenbildgebungsvorrichtung wäre ein solcher Parametervektor. Die/der Röntgenparameter 15 werden in FIG 2 mit dem C-Bogen-Gerät symbolisiert. Sie können, wie eingangs erwähnt, die Röhrenspannung beziehungsweise den Röhrenstrom, etwaige Winkelstellungen und Positionen oder dergleichen betreffen.

Basierend auf den Patienten- beziehungsweise Objektwerten 13 kann zumindest ein statistisches Patientenformmodell, aber auch ein statistisches Patientenorganmodell geschätzt werden. Auf der Grundlage von einem oder mehreren dieser Modelle und gegebenenfalls der System- beziehungsweise Röntgenparameter kann der Konfidenzwert abgeleitet werden.

Für die Ermittlung beziehungsweise Schätzung des Konfidenzwerts 12 können auch Kombinationen der oben genannten Objektwerte beziehungsweise Röntgenparameter verwendet werden.

Als Eingabeparameter kann auch ein erfasster 2D- oder 3D-Datensatz 16 des Objekts beziehungsweise Patienten 6 dienen. Auch dieser erfasste 2D- oder 3D-Datensatz 16 kann als Objektwert interpretiert werden. Er gibt Aufschluss über die konkrete Gestalt beziehungsweise Anatomie des Objekts. Ein solcher 2D- oder 3D-Datensatz 16 kann beispielsweise präoperativ oder sogar intraoperativ gewonnen werden. Während der Operation kann dann beispielsweise die CBCT-Aufnahme 17 mit den gewählten Röntgenparametern erfolgen. Unter Umständen kann es auch vorteilhaft sein, selbst nach der CBCT-Aufnahme 17 den 2D- oder 3D-Datensatz 16 beispielsweise für Referenzzwecke zu erfassen. Diese Datensätze können beispielsweise mit einem Spiral-CT-System gewonnen werden, das eine verhältnismäßig hohe Auflösung und Güte zeigt.

Alternativ oder zusätzlich kann auch eine frühere Angiographie- oder CBCT-Aufnahme 17 als Eingabeparameter für das Empfehlungssystem 11 zur Ermittlung des Konfidenzwerts 12 dienen. Solche früheren Aufnahmen können als Referenzwert dienen, um den Konfidenzwert zuverlässiger zu ermitteln.

Das Empfehlungssystem 11 kann auf unterschiedlichen Technologien basieren. Im einfachsten Fall wird eine Look-Up-Tabelle (LUT) verwendet, um den Eingangsparametern einen Ausgangswert (Konfidenzwert) zuzuordnen. Eine derartige Look-Up-Tabelle ist jedoch unflexibel. Eine höhere Flexibilität im Hinblick auf die Zuordnung eines oder mehrerer Konfidenzwerte zu einem Satz Eingangsparameter erhält man durch eine parametrisierbare Funktion. Die Zuordnungsvorschrift kann dabei selbst durch Parameter verändert werden. Diese Parameter können entweder manuell oder automatisch angepasst werden. Eine automatische Anpassung kann beispielsweise dazu genutzt werden, die Konfidenzwerte so zu spreizen, sodass ein gesamter, gewünschter Werteumfang genutzt wird.

Eine noch höhere Flexibilität bei der Zuordnung von Konfidenzwerten zu den Eingangsparametern erhält man bei Nutzung von Algorithmen, die maschinell lernen. Derartige Algorithmen können auf Gradient Boosting, neuronale Netze, neuronale Faltungsnetze, Transformatoren, Verstärkungslernen, große Sprachmodelle (LLMs) und so weiter gestützt werden. Eingangsgrößen eines solchen Algorithmus wären die Eingabeparameter und Ausgangsgröße der Konfidenzwert beziehungsweise die Konfidenzwertverteilung.

Bei einer vorteilhaften Ausführungsform kann vorgesehen sein, dass die Schwere verschiedener Artefakte separat angegeben wird. So ist beispielsweise die Streustrahlung im Bauraum stärker ausgeprägt als im Schädelbereich. Darüber hinaus können Empfehlungen zur Verbesserung der ermittelten Konfidenzwerte angegeben werden. So kann beispielsweise empfohlen werden, Kollimatoren zu nutzen, um die Streustrahlung zu reduzieren.

Bei einer weiteren vorteilhaften Ausgestaltung kann der diagnostische Konfidenzwert gleich zu Beginn eines Eingriffs geschätzt werden, wenn etwa im Voraus bekannt ist, dass eine bestimmte CBCT-Anwendung während des Eingriffs erforderlich ist.

Mit den erfindungsgemäßen Ansätzen ergibt sich der Vorteil, dass gegebenenfalls eine Röntgendosis eingespart werden kann, wenn der oder die ermittelten Konfidenzwerte einen Bediener veranlassen, beispielsweise das resultierende CBCT nicht durchzuführen. Darüber hinaus kann gegebenenfalls der Workflow (z.B. bei Kontrastmittelgabe) verbessert werden, da der Transfer z.B. zum CT entsprechend geplant werden kann. Ein weiterer Vorteil der Erfindung besteht darin, dass ein Radiologe ein objektives Modell zur Hand bekommt, wenn er die intra- oder postoperative Behandlung auf der Grundlage eines diagnostischen (CB-) CT plant.

## Patentansprüche

1. Verfahren zum Betreiben einer Röntgenbildgebungsvorrichtung durch
- Eingeben eines Objektwerts (13) betreffend ein zu durchleuchtendes Objekt (6) in die Röntgenbildgebungsvorrichtung und
- Einstellen eines Röntgenparameters (15) der Röntgenbildgebungsvorrichtung,
- Ermitteln eines Konfidenzwerts (12) bezüglich einer Qualität eines aufzunehmenden oder zu rekonstruierenden Röntgenbilds mit Hilfe eines Modells auf der Basis des Objektwerts (13) und des Röntgenparameters (15) und
- Bereitstellen des Konfidenzwerts (12) für einen Bediener oder zum Steuern der Röntgenbildgebungsvorrichtung,
wobei ein Wertebereich von Hounsfield-Werten einem Gewebetyp oder Objektbereich (14) zugeordnet wird, für den Wertebereich der Konfidenzwert (12) ermittelt wird und somit mittelbar über den Wertebereich dem Gewebetyp oder Objektbereich (14) der Konfidenzwert (12) zugeordnet wird.

2. Verfahren nach Anspruch 1, wobei der Konfidenzwert (12) einer von vielen Konfidenzwerten (12) ist, von denen jeder für ein jeweiliges Pixel oder Voxel des aufzunehmenden oder zu rekonstruierenden Röntgenbilds ermittelt wird.

3. Verfahren nach einem der vorhergehenden Ansprüche, wobei der Konfidenzwert (12) ein für eine jeweilige Schicht des zu rekonstruierenden Röntgenbilds standardisierter Wert ist.

4. Verfahren nach einem der vorhergehenden Ansprüche, wobei das Bereitstellen des Konfidenzwerts (12) in einer Karte erfolgt, in der an jedem Pixelort oder Voxelort ein entsprechender Konfidenzwert (12) dargestellt wird.

5. Verfahren nach einem der vorhergehenden Ansprüche, wobei bei dem Ermitteln des Konfidenzwerts (12) ein Qualitätsmaß bezüglich einer Rekonstruierbarkeit eines Voxels in Abhängigkeit von einer Detektortrajektorie der Röntgenbildgebungsvorrichtung berücksichtigt wird.

6. Verfahren nach einem der vorhergehenden Ansprüche, wobei das zu durchleuchtende Objekt (6) ein Patient ist und der Objektwert (13) mindestens einen der folgenden Werte betrifft: Größe, Gewicht, Geschlecht, Wasserwert oder anatomische Region des Patienten.

7. Verfahren nach einem der vorhergehenden Ansprüche, wobei der Objektwert (13) einen 3D-Datensatz (16) umfasst, der von dem Objekt (6) gewonnen wird, und ein jeweiliger Konfidenzwert (12) für jedes Voxel des 3D-Datensatzes (16) ermittelt wird.

8. Verfahren nach einem der vorhergehenden Ansprüche, wobei dem Ermitteln des Konfidenzwerts (12) ein statistisches Objektmodell zu Grunde gelegt wird, das mit dem Objektwert (13) parametrisiert wird.

9. Verfahren nach einem der vorhergehenden Ansprüche, wobei der Röntgenparameter (15) der Röntgenbildgebungsvorrichtung mindestens eine der folgenden Werte betrifft: Röhrenspannung, Röhrenstrom, Detektorwinkel oder Detektorposition.

10. Verfahren nach einem der vorhergehenden Ansprüche, wobei das Ermitteln des Konfidenzwerts (12) mit Hilfe einer Look-Up-Tabelle, einer parametrierbaren Funktion oder eines Algorithmus zum maschinellen Lernen erfolgt.

11. Verfahren nach einem der vorhergehenden Ansprüche, wobei in Abhängigkeit von dem bereitgestellten Konfidenzwert (12) ein Parameter der Röntgenbildgebungsvorrichtung automatisch eingestellt wird.

12. Steuerungseinrichtung (7) für eine Röntgenbildgebungsvorrichtung mit
- einer ersten Schnittstelleneinrichtung zum Eingeben eines Objektwerts (13) betreffend ein zu durchleuchtendes Objekt (6) in die Steuerungseinrichtung (7) und zum Einstellen eines Röntgenparameters (15) der Röntgenbildgebungsvorrichtung,
**gekennzeichnet dadurch**,
- einer Recheneinrichtung (8) ausgebildet zum Ermitteln eines Konfidenzwerts (12) bezüglich einer Qualität eines aufzunehmenden Röntgenbilds mit Hilfe eines Modells auf der Basis des Objektwerts (13) und des Röntgenparameters (15) und
- einer zweiten Schnittstelleneinrichtung zum Bereitstellen des Konfidenzwerts (12) für einen Bediener oder zum Steuern der Röntgenbildgebungsvorrichtung, wobei ein Wertebereich von Hounsfield-Werten einem Gewebetyp oder Objektbereich (14) zugeordnet wird, für den Wertebereich der Konfidenzwert (12) ermittelt wird und somit mittelbar über den Wertebereich dem Gewebetyp oder Objektbereich (14) der Konfidenzwert (12) zugeordnet wird.

13. Röntgenbildgebungsvorrichtung mit einer Röntgenbildgebungseinheit und einer Steuerungseinrichtung (7) nach Anspruch 12, die zum Steuern der Röntgenbildgebungseinheit ausgebildet ist.

## Claims

1. Method for operating an X-ray imaging apparatus by
- inputting to the X-ray imaging apparatus an object value (13) relating to an object (6) to be X-rayed, and
- configuring an X-ray parameter (15) of the X-ray imaging apparatus,
- determining by means of a model, on the basis of the object value (13) and the X-ray parameter (15), a confidence value (12) regarding a quality of an X-ray image to be acquired or reconstructed, and
- providing the confidence value (12) for an operator or for the control of the X-ray imaging apparatus,
wherein a value range of Hounsfield values is assigned to a tissue type or object region (14), the confidence value (12) is determined for the value range, and the confidence value (12) is thereby assigned indirectly via the value range to the tissue type or object region (14).

2. Method according to claim 1, wherein the confidence value (12) is one of many confidence values (12), each of which is determined for a particular pixel or voxel of the X-ray image to be acquired or reconstructed.

3. Method according to one of the preceding claims, wherein the confidence value (12) is a value standardised for a particular slice of the X-ray image to be reconstructed.

4. Method according to one of the preceding claims, wherein the providing of the confidence value (12) is made in a map in which at each pixel location or voxel location is presented a corresponding confidence value (12).

5. Method according to one of the preceding claims, wherein the determining of the confidence value (12) takes into account a quality metric regarding a reconstructibility of a voxel according to a detector trajectory of the X-ray imaging apparatus.

6. Method according to one of the preceding claims, wherein the object (6) to be X-rayed is a patient, and the object value (13) relates to at least one of the following values: size, weight, gender, water equivalent value or anatomical region of the patient.

7. Method according to one of the preceding claims, wherein the object value (13) comprises a 3D dataset (16), which is obtained from the object (6), and an associated confidence value (12) is determined for each voxel of the 3D dataset (16).

8. Method according to one of the preceding claims, wherein the determining of the confidence value (12) is based on a statistical object model, which is parameterised by the object value (13).

9. Method according to one of the preceding claims, wherein the X-ray parameter (15) of the X-ray imaging apparatus relates to at least one of the following values: tube voltage, tube current, detector angle, or detector position.

10. Method according to one of the preceding claims, wherein the determining of the confidence value (12) is performed with the aid of a lookup table, a parameterisable function or a machine learning algorithm.

11. Method according to one of the preceding claims, wherein a parameter of the X-ray imaging apparatus is configured automatically according to the confidence value (12) provided.

12. Control facility (7) for an X-ray imaging apparatus having
- a first interface facility for inputting to the control facility (7) an object value (13) that relates to an object (6) to be X-rayed and for configuring an X-ray parameter (15) of the X-ray imaging apparatus,
**characterised by**
- a computing facility (8) designed to determine by means of a model, on the basis of the object value (13) and the X-ray parameter (15), a confidence value (12) regarding a quality of an X-ray image to be acquired, and
- a second interface facility for providing the confidence value (12) for an operator or for the control of the X-ray imaging apparatus, wherein a value range of Hounsfield values is assigned to a tissue type or object region (14), the confidence value (12) is determined for the value range, and the confidence value (12) is thereby assigned indirectly via the value range to the tissue type or object region (14).

13. X-ray imaging apparatus having an X-ray imaging unit and a control facility (7) according to claim 12, which is designed to control the X-ray imaging unit.

## Revendications

1. Procédé pour faire fonctionner une installation d'imagerie par rayons X par
- entrée d'une valeur (13) d'objet concernant un objet (6) à éclairer dans l'installation d'imagerie par rayons X et
- réglage d'un paramètre (15) de rayons X de l'installation d'imagerie par rayons X,
- détermination d'une valeur (12) de confiance en ce qui concerne une qualité d'une image de rayons X à enregistrer ou à reconstruire à l'aide d'un modèle sur la base de la valeur (13) d'objet et du paramètre (15) de rayons X et
- mise à disposition de la valeur (12) de confiance pour un opérateur ou pour la commande de l'installation d'imagerie par rayons X,
dans lequel une plage de valeurs de Hounsfield est affectée à un type de tissu ou à une zone (14) de l'objet, on détermine la valeur (12) de confiance pour la plage de valeurs et on affecte ainsi indirectement par la plage de valeurs la valeur (12) de confiance au type de tissu ou à la zone (14) de l'objet.

2. Procédé suivant la revendication 1, dans lequel la valeur (12) de confiance est l'une de plusieurs valeurs (12) de confiance, dont chacune est déterminée pour un pixel ou un voxel respectif de l'image de rayons X à enregistrer ou à reconstruire.

3. Procédé suivant l'une des revendications précédentes, dans lequel la valeur (12) de confiance est une valeur normalisée pour une couche respective de l'image de rayons X à reconstruire.

4. Procédé suivant l'une des revendications précédentes, dans lequel la mise à disposition de la valeur (12) de confiance s'effectue dans une carte, dans laquelle il est représenté, sur chaque emplacement de pixel ou emplacement de voxel, une valeur (12) de confiance correspondante.

5. Procédé suivant l'une des revendications précédentes, dans lequel, lors de la détermination de la valeur (12) de confiance, on prend en compte une mesure de qualité en ce qui concerne une possibilité de reconstruction d'un voxel en fonction d'une trajectoire de détecteur de l'installation d'imagerie par rayons X.

6. Procédé suivant l'une des revendications précédentes, dans lequel l'objet (6) à éclairer est un patient et la valeur (13) de l'objet concerne au moins l'une des valeurs suivantes : taille, poids, sexe, valeur en eau ou région anatomique du patient.

7. Procédé suivant l'une des revendications précédentes, dans lequel la valeur (13) d'objet comprend un ensemble (16) de données en 3D, que l'on obtient à partir de l'objet (6), et on détermine une valeur (12) de confiance respective pour chaque voxel de l'ensemble (16) de données en 3D.

8. Procédé suivant l'une des revendications précédentes, dans lequel un modèle d'objet statistique, que l'on paramétrise par la valeur (13) d'objet, est à la base de la détermination de la valeur (12) de confiance.

9. Procédé suivant l'une des revendications précédentes, dans lequel le paramètre (15) de rayons X de l'installation d'imagerie par rayons X conserne au moins l'une des valeurs suivantes : tension de tube, courant de tube, angle détecteur ou position du détecteur.

10. Procédé suivant l'une des revendications précédentes, dans lequel la détermination de la valeur (12) de confiance s'effectue à l'aide d'une table de consultation, d'une fonction paramétrable ou d'un algorithme pour l'apprentissage par machine.

11. Procédé suivant l'une des revendications précédentes, dans lequel on établit automatiquement un paramètre de l'installation d'imagerie par rayons X en fonction de la valeur (12) de confiance mise à disposition.

12. Dispositif (7) de commande d'une installation d'imagerie par rayons X comprenant
- un premier dispositif d'interface pour l'entrée d'une valeur (13) d'objet concernant un objet (6) à éclairer dans le dispositif (7) de commande et pour le réglage d'un paramètre (15) de rayons X de l'installation d'imagerie par rayons X, **caractérisé par**
- un dispositif (8) informatique constitué pour la détermination d'une valeur (12) de confiance en ce qui concerne une qualité d'une image de rayons X à enregistrer à l'aide d'un modèle sur la base de la valeur (13) d'objet et du paramètre (15) de rayons X, et
- un deuxième dispositif d'interface pour mettre la valeur (12) de confiance à disposition d'un opérateur ou pour la commande de l'installation d'imagerie par rayons X, dans lequel on affecte une plage de valeurs de Hounsfield à un type de tissu ou à une zone (14) de l'objet, on détermine, pour la plage de valeurs, la valeur (12) de confiance et on affecte ainsi indirectement par la plage de valeurs la valeur (12) de confiance au type de tissu ou à la zone (14) d'objet.

13. Installation d'imagerie par rayons X comprenant une unité d'imagerie par rayons X et un dispositif (7) de commande suivant la revendication 12, qui est constituée pour la commande de l'unité d'imagerie par rayons X.
